# EUROPEAN PATENT APPLICATION

(11) **EP 2 389 882 A1**
(43) Date of publication of application: **30.11.2011**
(21) Application number: 09838637.8
(22) Date of filing: 02.07.2009
(51) Int. Cl.: A61B 17/34

(54) **GROOVE UNIVERSAL RADIAL SEAL RING FOR PUNCTURE DEVICE AND PUNCTURE DEVICE**

(30) Priority: 23.01.2009 CN 200910036989
(71) Applicant: Zhou, Xing, Guangzhou, Guangdong 510060 (CN); Li, Yanfang, Guangdong 510060 (CN)
(72) Inventor: Zhou, Xing, Guangzhou, Guangdong 510060 (CN); Li, Yanfang, Guangdong 510060 (CN)
(74) Representative: Palmer, Jonathan R.
(86) International application number: PCT/CN2009/072595
(87) International publication number: WO 2010/083670

(57) **Abstract**

A grooved universal radial seal ring used in a trocar and a trocar are provided. The radial seal ring includes one or more easily-deformable grooves, one or more supporting areas capable of guiding surgical device movement, and a through hole, the supporting areas of the seal ring are connected by the easily-deformable grooves, a thickness of the easily-deformable grooves is much smaller than an average thickness of the supporting areas, so that when a surgical device is inserted along a cone-shaped guiding groove of the supporting areas, the easily-deformable grooves deform first and surround the surgical device because a wall thickness of the easily-deformable grooves is very small, thereby achieving a sealing effect. When the surgical device shakes, the supporting areas separated by the easily-deformable grooves also shake together, but the seal ring formed by the easily-deformable grooves still surrounds the surgical device, thereby always keeping a sealed state and achieving an excellent dynamic sealing effect. Particularly, the thickness of the easily-deformable grooves is small, not only a dynamic sealing effect is excellent, but also surgical device moving resistance is small. The trocar realizes any replacement among surgical devices with diameters being 12 millimeters (mm), 10 mm, and 5 mm, thereby bringing convenience to surgical operation.

## Description

### BACKGROUND OF THE INVENTION

### Field of the Invention

The present invention relates generally to a medical device, and more particularly to a trocar used in minimally invasive laparoscopic surgery and a seal ring used in the trocar.

### Related Art

In modern medicine, the minimally invasive laparoscopic surgery is widely applied. In order to avoid iatrogenic infection, disposable trocars are used in the laparoscopic surgery, so that an improvement direction of the trocar is to simplify its structure and decrease its cost without adversely affecting its performance.

In the prior art, a seal structure used in a trocar includes a radial seal ring and a one-way valve. The one-way valve can be classified into two categories. One includes a spring, a blocking plate, and a silica gel seal ring. The spring is configured to press the blocking plate against the silica gel seal ring to seal. The spring and the blocking plate are usually made of stainless steel. The seal ring of this form is often used in a non-disposable trocar made of metal, which is expensive and heavy. In recent years, people have developed a funnel-shaped silica gel seal ring to be used by disposable trocars. The funnel-shaped silica gel seal ring has a straight through-hole incision at its bottom, and a sealing effect is achieved by the silica gel's contraction force and a pressure formed by carbon dioxide pneumoperitoneum. The funnel-shaped silica gel seal ring is widely used in a disposable trocar. The radial seal ring has a ring-shaped structure having a center hole, and specifically has a thickness decreasing gradually from an outer edge to the center hole. The structure with the thickness decreasing gradually from the outer edge to the center hole is prone to leaking when a surgical device shakes, resulting in a poor dynamic sealing effect. For a prior art radial seal ring, a conversion sheath is required when a 5 millimeters (mm) surgical device is used in a 10 mm trocar, which causes inconvenience to surgical operation performed by a doctor.

### SUMMARY OF THE INVENTION

### Technical problem

The present invention is directed to a universal radial seal ring used in a trocar, so that both a 10 mm surgical device and a 5 mm surgical device can be used in a 10 mm trocar without requiring a conversion sheath. The radial seal ring has a delicate structure, and it is highly reliable and cost-effective, and achieves an excellent sealing effect.

One key idea of the technical solutions of the present invention is as follows: a radial seal ring is configured with one or more easily deformable grooves having a very small thickness, so that the moving resistance encountered by a surgical device inserted therein is small. The supporting areas of the seal ring separated by the one or more easily deformable grooves not only have suitable strength to prevent flip of the seal ring when the surgical device is withdrawn, but also always surrounds the surgical device as the surgical device shakes, thereby achieving a sealing effect.

### Technical solution

A grooved universal radial seal ring used in a trocar according to the present invention includes one or more easily-deformable grooves, one or more movement-guiding supporting areas, and a through hole. The supporting areas are connected by the easily-deformable grooves. The edges of the easily-deformable grooves are connected to the through hole. A maximum thickness of the supporting areas is at least twice as much as a thickness δ of the easily-deformable grooves. Because the wall thickness of the easily-deformable grooves is very small, when a surgical device is inserted along a funnel-shaped guiding groove of the supporting areas, it causes the easily-deformable grooves to deform and surround the surgical device, thereby achieving a sealing effect. When the surgical device shakes, the supporting areas separated by the easily-deformable grooves also shake together, but the seal ring formed by the easily-deformable grooves still surrounds the surgical device, thereby keeping a sealed state and achieving an excellent dynamic sealing effect. Particularly, the thickness δ of the easily-deformable grooves is small, which not only improves the dynamic sealing effect, but also reduces the surgical device's moving resistance, which makes it convenient for operation performed by a doctor.

Furthermore, the thickness δ of the easily-deformable grooves ranges from 0.1 mm to 1.2 mm, and a preferred value of the thickness δ ranges from 0.3 mm to 0.7 mm.

The seal ring has two or more easily-deformable grooves, evenly distributed on the seal ring. For example, three, four, five, six, or seven easily-deformable grooves are evenly distributed on the seal ring. The seal ring that has four or five easily-deformable grooves has the optimal sealing effect.

A diameter of the through hole ranges from 3 mm to 4 mm, and a preferred value ranges from 3.5 mm to 4.2 mm.

A movement-guiding angle α of the supporting areas ranges from 40° to 80°, and a preferred value ranges from 55° to 75°.

The supporting areas capable of guiding surgical device movement of the seal ring are covered with a coating of super-slippery material. The super-slippery material coating is usually made of a hydrophilic material, and may also be made of a material having a self-lubricating function.

The seal ring is made of a medical elastic material, including, but not limited to, medical silica gel, medical rubber, medical polyurethane, medical latex, and a combination thereof. The seal ring may be made of one or multiple materials.

The present invention also provides a trocar, adopting the grooved universal radial seal ring according to the present invention.

A fixing rack of a one-way valve of the trocar includes a support surface meeting a funnel surface of the one-way valve, and the funnel surface of the one-way valve is closely attached to the support surface on the fixing rack. The support surface on the fixing rack can effectively keep the shape of the funnel surface of the one-way valve, thereby improving sealing performance of the one-way valve, especially the sealing performance after a device moves.

### Improvements

The radial seal ring of the present invention includes one or more easily-deformable grooves, one or more supporting areas capable of guiding surgical device movement, and a through hole. The supporting areas of the seal ring are connected by the easily-deformable grooves, and the thickness of the easily-deformable grooves is much smaller than an average thickness of the supporting areas, so that when a surgical device is inserted along a funnel-shaped guiding groove of the supporting areas, the easily-deformable grooves deform first and surround the surgical device because the wall thickness of the easily-deformable grooves is very small, thereby achieving the sealing effect. When the surgical device shakes, the supporting areas separated by the easily-deformable grooves also shake together, but the seal ring formed by the easily-deformable grooves still surrounds the surgical device, thereby always keeping a sealed state and achieving an excellent dynamic sealing effect. Particularly, the thickness δ of the easily-deformable grooves is small to not only improve the dynamic sealing effect, but also reduce the surgical device moving resistance, which makes it convenient for operation performed by a doctor. The grooved universal radial seal ring of the present invention takes into account of both the sealing effect and surgical device moving resistance, thereby eliminating the defect in the prior art that a radial sealing effect of a radial seal ring is achieved by having a large surgical device moving resistance and when the surgical device moving resistance is small, the radial sealing effect is poor. The seal ring of the present invention can be widely applied to trocars for laparoscopic surgery.

In addition, the cone of the supporting areas of the radial seal ring according to the present invention not only has a guiding function, but also has an anti-flip function when the surgical device is pulled out. The flip of the radial seal ring greatly increases resistance encountered by the surgical device being withdrawn. The seal ring of the present invention has the supporting areas to prevent flipping.

By incorporating the seal ring according to the present invention, any surgical device with the diameter being 10 mm or 5 mm can be inserted while maintaining excellent dynamic sealing performance. The structure allows any replacement between surgical devices with the diameters ranging from 12 mm, 10 mm, and 5 mm, any replacement between surgical devices with the diameters ranging from 15 mm, 12 mm, 10 mm, and 5 mm, and any replacement between surgical devices with the diameters ranging from 10 mm, 12 mm, and 15 mm.

### BRIEF DESCRIPTION OF THE DRAWINGS

FIG. 1 is a schematic structural view of a four-groove seal ring according to the present invention;
FIG. 2 is a schematic structural view of the back side of the seal ring of FIG. 1;
FIG. 3 is an A-A cross-sectional view of FIG. 1;
FIG. 4 is a B-B cross-sectional view of FIG. 1, in which δ is a thickness of easily-deformable grooves, and α is a guiding angle of a cone of supporting areas;
FIG. 5 is a schematic structural view of another four-groove seal ring according to the present invention;
FIG. 6 is a schematic structural view of the back side of the seal ring of FIG. 5;
FIG. 7 is a C-C cross-sectional view of FIG. 5;
FIG. 8 is a D-D cross-sectional view of FIG. 5;
FIG. 9 is a schematic structural view of a two-groove seal ring according to the present invention;
FIG. 10 is a schematic structural view of a three-groove seal ring according to the present invention;
FIG. 11 is a schematic structural view of a grooved seal ring with supporting areas having a super-slippery material coating;

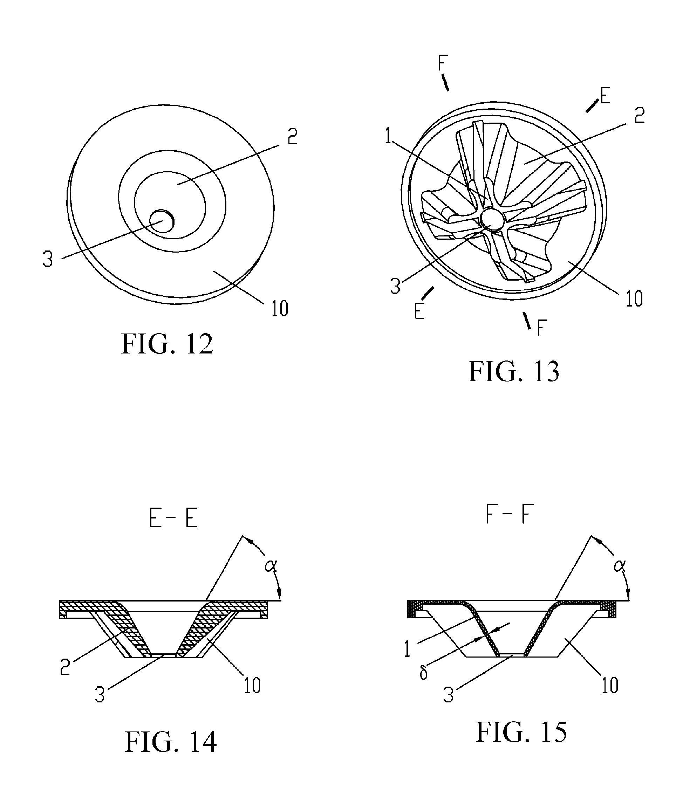

FIG. 12 is a schematic structural view of a front of a grooved seal ring with easily-deformable grooves disposed on a back;
FIG. 13 is a schematic structural view of the back of the grooved seal ring of FIG. 12;
FIG. 14 is an E-E cross-sectional view of FIG. 13;
FIG. 15 is an F-F cross-sectional view of FIG. 13;
FIG. 16 is an assembly view of a trocar according to the present invention, in which the trocar uses the seal ring according to the present invention;
FIG. 17 is a schematic structural view of a fixing rack of a one-way valve of the trocar of FIG. 16;
FIG. 18 is a G-G cross-sectional view of FIG. 17;
FIG. 19 is an H-H cross-sectional view of FIG. 17;
FIG. 20 is a schematic structural view of the one-way valve adopted by the trocar of FIG. 16;
FIG. 21 is a schematic structural view of the back side of FIG. 20;
FIG. 22 is a J-J cross-sectional view of FIG. 21;
FIG. 23 is a K-K cross-sectional view of FIG. 21;
FIG. 24 is a schematic structural view of the assembled trocar of FIG. 16 according to the present invention;
FIG. 25 is a schematic structural view of the trocar of FIG. 24 into which a surgical device is already inserted;
FIG. 26 is a schematic structural view of a five-groove universal radial seal ring according to the present invention, in which the grooved universal radial seal ring according to an embodiment includes five easily-deformable grooves;
FIG. 27 is top view of FIG. 26;
FIG. 28 is an M-M direction cross-sectional view of FIG. 27; and
FIG. 29 is a schematic structural view of a trocar according to the present invention, in which in an embodiment, the five-groove universal radial seal ring of FIG. 26 according to the present invention is adopted.

In figures: 1 represents an easily-deformable groove, 2 represents a supporting area capable of guiding surgical device movement, 3 represents a through hole,δ represents a thickness of the easily-deformable grooves, α represents a guiding angle of a cone of the supporting areas, 10 represents a seal ring according to the present invention, 11 represents an outer sheath of a trocar, 12 represents a one-way valve, 13 represents a fixing rack of the one-way valve, 14 represents a valve, 15 represents an upper cover, 16 represents a pressing member, 17 represents an anti-slip pattern on the outer sheath of the trocar, and 18 represents a surgical device. 21 represents a super-slippery material coating on the supporting areas. 121 represents an incision on the one-way valve, 122 represents a lateral strengthening rib on the one-way valve, 123 represents a protruding rib on the one-way valve, 124 represents a funnel surface on the one-way valve, 125 represents a reinforcement rib on the one-way valve, and 126 represents a vent hole on the one-way valve. 131 represents a support surface on the fixing rack of the one-way valve.

### DETAILED DESCRIPTION OF THE INVENTION

### Embodiment 1: a four-groove seal ring according to the present invention

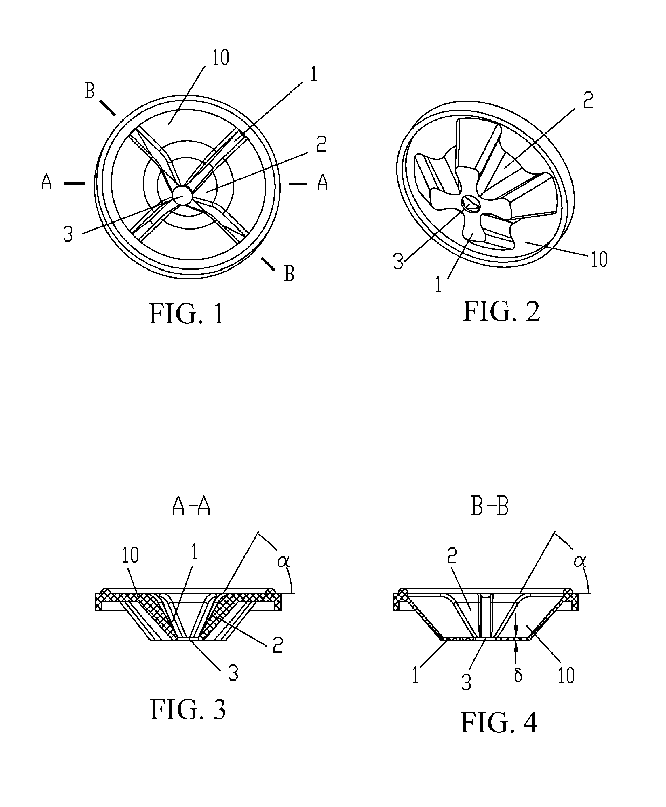

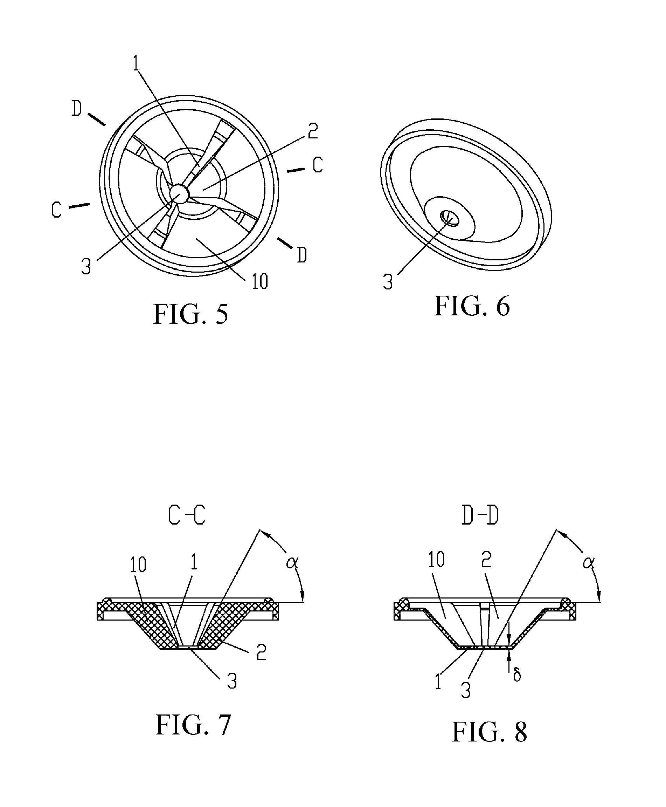

The part drawings are made according to technical solutions of the present invention. A center of a seal ring (10) of the present invention includes a through hole (3). The through hole (3) is for access of a surgical device. The seal ring (10) of the present invention includes four easily-deformable grooves (1). The supporting areas (2) capable of guiding surgical device movement are connected to the easily-deformable grooves (1) respectively. Reference is made to FIG. 1 to FIG. 8.

Highly tear-resistant medical silica gel is used to make the seal ring of the present invention, and the seal ring of the present invention can be made using a molding process of the medical silica gel.

A thickness δ of the easily-deformable grooves (1) is used for adjusting a sealing effect and surgical device moving resistance. The thickness δ of the easily-deformable grooves (1) ranges from 0.1 mm to 1.2 mm, and an optimal value ranges from 0.3 mm to 0.7 mm. In addition, a width and a shape of the easily-deformable grooves (1) may also affect the sealing effect and the surgical device moving resistance to some extent, but the thickness δ is a key parameter.

Changing the shape and the wall thickness of the supporting areas (2) capable of guiding surgical device movement may also affect the sealing effect and the surgical device moving resistance. Normally, a maximum thickness of the supporting areas (2) is at least twice as much as the thickness δ of the easily-deformable grooves (1), and is preferably 3 to 4.5 times of the thickness δ of the easily-deformable grooves (1). A guiding angle α of a cone of the supporting areas (2) capable of guiding surgical device movement may also affect the sealing effect and the surgical device moving resistance. The guiding angle α may range from 40° to 80°, and an optimal value ranges from 55° to 75°.

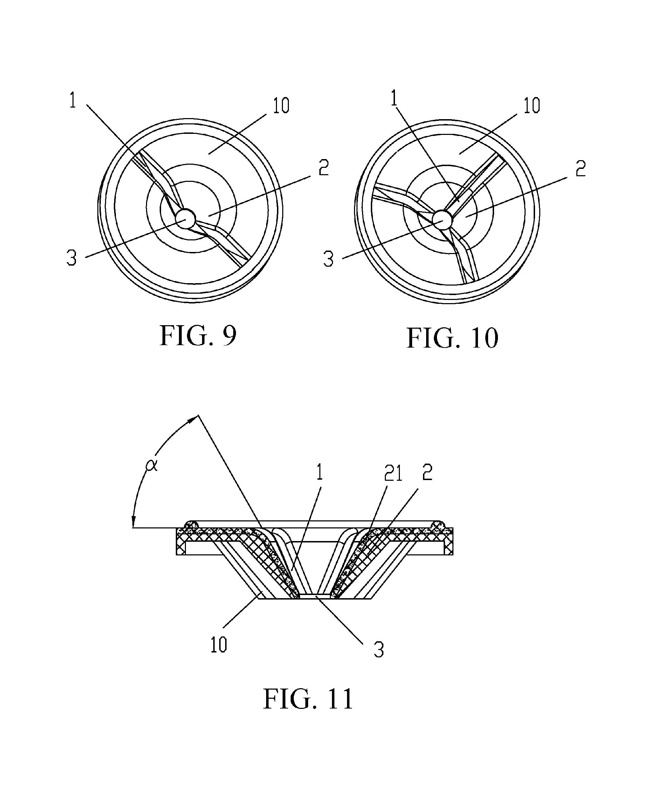

In addition, the seal ring of the present invention may be made of any medical elastic material, including, but not limited to, medical silica gel, medical rubber, medical polyurethane, medical latex, and a combination thereof. For example, the supporting areas (2) are made of medical silica gel of high hardness, and the easily-deformable grooves (1) are made of highly tear-resistant medical silica gel of low hardness. Particularly, a surface of the guiding cone of the supporting areas (2) may be processed by, for example, adding a super-slippery material coating (21), which can significantly reduce the surgical device moving resistance and keep the excellent sealing effect. Reference is made to FIG. 11.

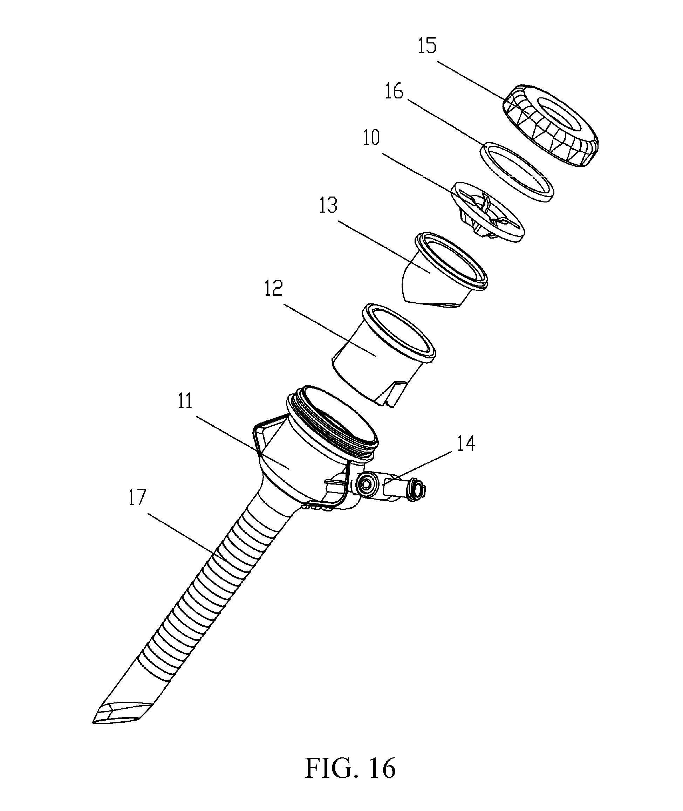

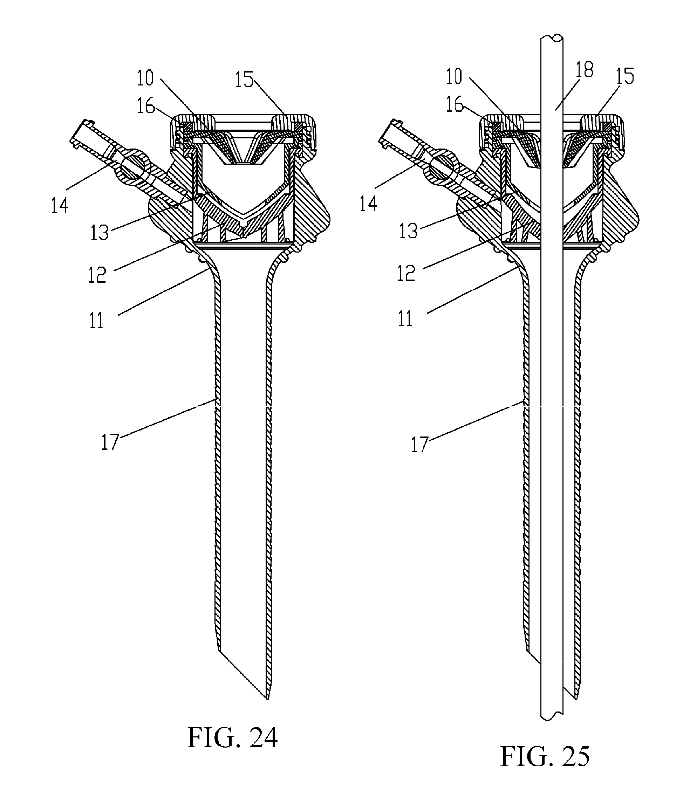

The seal ring (10) of the present invention includes the easily-deformable grooves (1), the supporting areas (2) capable of guiding surgical device movement, and the through hole (3), the supporting areas (2) are connected by the easily-deformable grooves (1), the thickness δ of the easily-deformable grooves is much smaller than an average thickness of the supporting areas (2), so that when a surgical device (18) is inserted along a cone-shaped guiding groove of the supporting areas (2), the easily-deformable grooves (1) deform first and surround the surgical device (18) because the wall thickness of the easily-deformable grooves (1) is very small, thereby achieving the sealing effect. When the surgical device (18) shakes, the supporting areas (2) separated by the easily-deformable grooves also shake together, but the seal ring formed by the easily-deformable grooves (1) still surrounds the surgical device (18), thereby always keeping a sealed state and achieving an excellent dynamic sealing effect. Particularly, the thickness δ of the easily-deformable grooves is small, not only the dynamic sealing effect is excellent, but also the surgical device moving resistance is small, which makes it convenient for operation performed by a doctor. The grooved universal radial seal ring (10) of the present invention takes into account both the sealing effect and the surgical device moving resistance, which are harmoniously combined, thereby eliminating the defect in the prior that when the radial sealing effect of a radial seal ring is excellent, the surgical device moving resistance is large; while when the surgical device moving resistance is small, the radial sealing effect is poor. The seal ring of the present invention can be widely applied to trocars for laparoscopic surgery. Reference is made to FIG. 16, FIG. 24, and FIG. 25.

In addition, the guiding part of the supporting areas (2) of the radial seal ring of the present invention takes form of a cone, thereby not only having a guiding function, but also having an anti-flip function when the surgical device is withdrawn to the outside. The flip of the radial seal ring greatly increases resistance encountered by the surgical device being withdrawn. The seal ring of the present invention has the supporting areas (2), so that no flip occurs.

By adopting the seal ring according to the present invention, any surgical device with the diameter being 10 mm or 5 mm can be inserted, and excellent dynamic sealing performance is kept.

The structure can also realize any replacement among surgical devices with the diameters being 12 mm, 10 mm, and 5 mm, any replacement among surgical devices with the diameters being 15 mm, 12 mm, 10 mm, and 5 mm, and any replacement among surgical devices with the diameters being 10 mm, 12 mm, and 15 mm, the key of which lies in the setting of the suitable thickness δ of the easily-deformable grooves and the suitable diameter of the through hole (3).

### Embodiment 2: a two-groove seal ring according to the present invention

The technical principle of this embodiment is the same as that of Embodiment 1, and differences only lie in that the seal ring only includes two easily-deformable grooves (1), and the two easily-deformable grooves (1) are connected in sequence to two supporting areas (2) capable of guiding surgical device movement.

### Embodiment 3: a three-groove seal ring according to the present invention

The technical principle of this embodiment is the same as that of Embodiment 1, and differences only lie in that the seal ring only includes three easily-deformable grooves (1), and the three easily-deformable grooves (1) are connected in sequence to three supporting areas (3) capable of guiding surgical device movement.

### Embodiment 4: a four-groove seal ring according to the present invention with inversely disposed easily-deformable grooves

The technical principle of this embodiment is the same as that of Embodiment 1, and differences only lie in that only the back of the seal ring includes four easily-deformable grooves (1), the four easily-deformable grooves (1) are connected in sequence to four supporting areas (4) capable of guiding surgical device movement, and the easily-deformable grooves (1) cannot be seen from the front of the seal ring.

### Embodiment 5: a five-groove seal ring according to the present invention

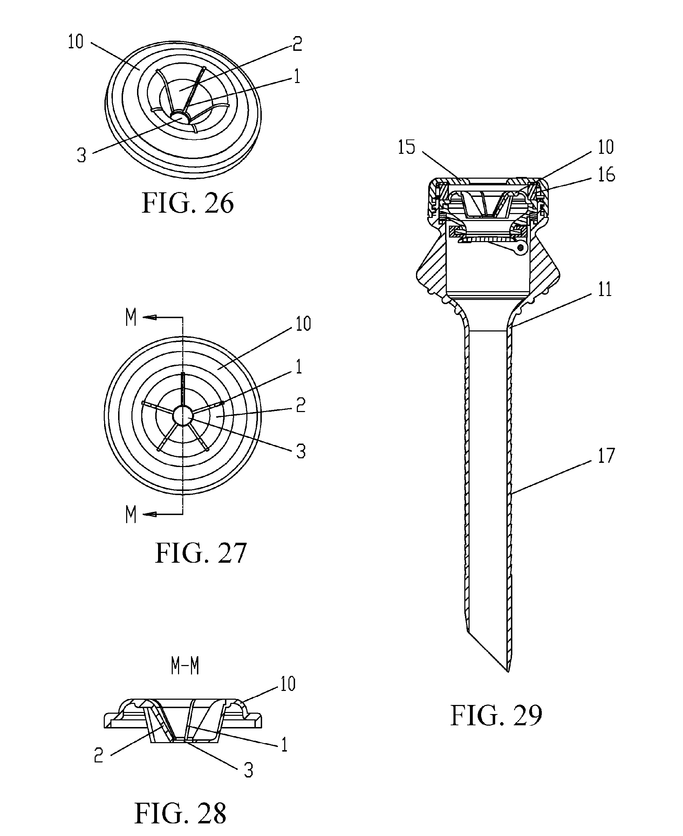

The technical principle of this embodiment is the same as that of Embodiment 1, and differences only lie in that the seal ring only includes five easily-deformable grooves (1), and the five easily-deformable grooves (1) are connected in sequence to five supporting areas (5) capable of guiding surgical device movement. Reference is made to FIG. 26 to FIG. 29.

The seal ring of the present invention includes the five easily-deformable grooves (1), so that a groove width of each of the easily-deformable grooves (1) is small, thereby making it easy for different surgical device to pass by.

### Embodiment 6: a trocar according to the present invention

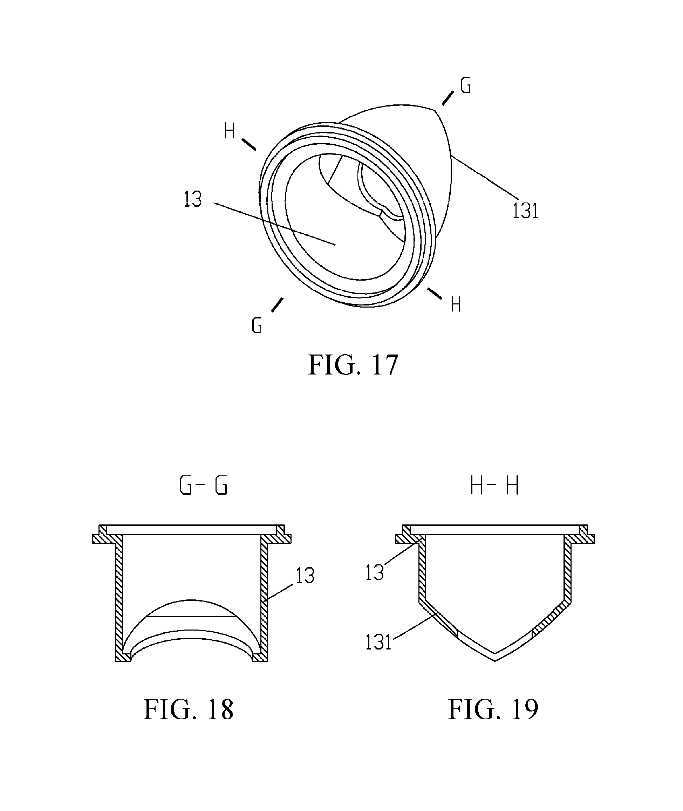

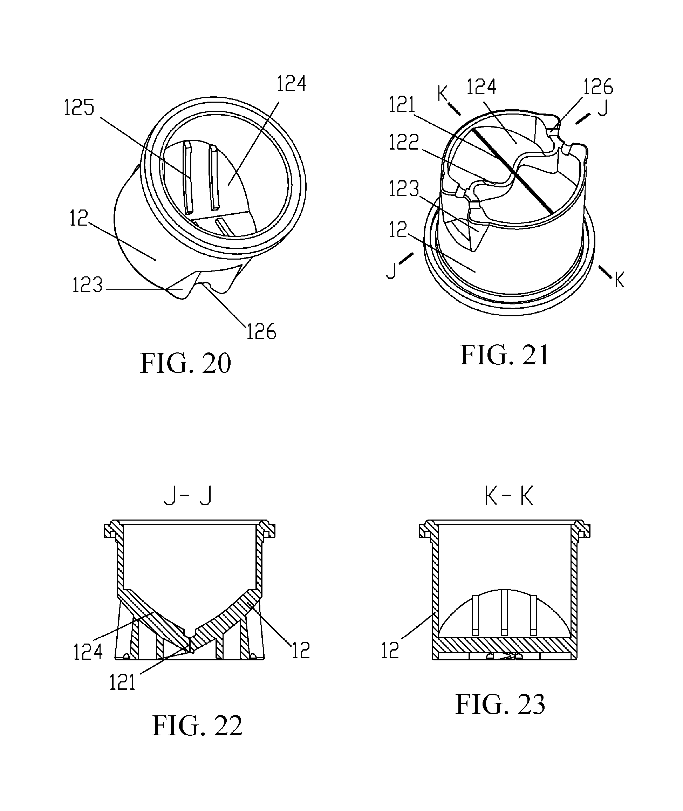

The seal ring (10), a one-way valve (12), an outer sheath (11) of the trocar, a fixing rack (13) of the one-way valve, a valve (14), an upper cover (15), and a pressing member (16) according to the present invention are manufactured in sequence. The seal ring (10) according to the present invention and the one-way valve (12) are made of medical rubber. The outer sheath (11) of the trocar, the fixing rack (13) of the one-way valve, the valve (14), the upper cover (15), and the pressing member (16) are made of plastic by injection molding. An anti-slip pattern (17) on the outer sheath (11) of the trocar may be directly formed on the outer sheath (11) of the trocar by injection molding. Assembly is performed in sequence according to the design, thereby acquiring the trocar according to the present invention. Reference is made to FIG. 16 to FIG. 25.

It should be noted that, the structures disclosed and described herein may be replaced with other structures of the same effect, and meanwhile embodiments of the present invention described herein are not the sole structure to implement the present invention. Although preferred embodiments of the present invention are illustrated herein, it should be understood by persons skilled in the art that the embodiments are only for illustrating, and various modifications, improvements, and replacements may be made by persons skilled in the art without departing from the present invention. Therefore, the protection scope of the present invention is defined according to the spirit and scope of the appended claims of the present invention.

## Claims

1. A grooved universal radial seal ring used in a trocar, wherein the seal ring includes one or more easily-deformable grooves (1), one or more supporting areas (2) capable of guiding surgical device movement, and a through hole (3), the supporting areas (2) are connected by the easily-deformable grooves (3), edges of the easily-deformable grooves (1) are connected to the through hole (3), and a maximum thickness of the supporting areas (2) is at least twice as much as a thickness δ of the easily-deformable grooves (1).

2. The grooved universal radial seal ring used in a trocar according to claim 1,
wherein the thickness δ of the easily-deformable grooves (1) ranges from 0.1 millimeter (mm) to 1.2 mm, and an optimal value of the thickness δ ranges from 0.3 mm to 0.7 mm.

3. The grooved universal radial seal ring used in a trocar according to claim 1,
wherein the seal ring includes two or more easily-deformable grooves (1), evenly distributed on the seal ring.

4. The grooved universal radial seal ring used in a trocar according to claim 1,
wherein a guiding angle α of the supporting areas (2) capable of guiding surgical device movement of the seal ring ranges from 40° to 80°, and an optimal value ranges from 55° to 75°.

5. The grooved universal radial seal ring used in a trocar according to claim 1,
wherein the seal ring is made of a medical elastic material, comprising, but not limited to, medical silica gel, medical rubber, medical polyurethane, medical latex, and a combination thereof.

6. The grooved universal radial seal ring used in a trocar according to claim 1,
wherein the supporting areas (2) capable of guiding surgical device movement of the seal ring are disposed with a super-slippery material coating (21).

7. The grooved universal radial seal ring used in a trocar according to claim 1,
wherein a diameter of the through hole (3) ranges from 3 mm to 4.8 mm, and an optimal value ranges from 3.5 mm to 4.2 mm.

8. A trocar, comprising the grooved universal radial seal ring according to claim 1.

9. The trocar according to claim 8, wherein a fixing rack (13) of a one-way valve of the trocar includes a support surface (131) matching a funnel surface (124) of the one-way valve (12).
